(19) Europäisches Patentamt
European Patent Office
Office européen des brevets

(11) EP 3 483 248 A1

(12) **EUROPEAN PATENT APPLICATION**

(43) Date of publication:
**15.05.2019 Bulletin 2019/20**

(21) Application number: **18178753.2**

(22) Date of filing: **20.06.2018**

(51) Int Cl.:
***C11D 3/38*** *(2006.01)* ***C07K 14/46*** *(2006.01)*
***C07K 14/47*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.11.2017 EP 17201322**

(71) Applicant: **The Procter & Gamble Company**
**Cincinnati, OH 45202 (US)**

(72) Inventors:
- **BETTIOL, Jean-Luc Philippe**
**1853 Strombeek-Bever (BE)**
- **GONZALES, Denis Alfred**
**1853 Strombeek-Bever (BE)**
- **VELASQUEZ, Juan Esteban**
**Cincinnati, Ohio 45202 (US)**
- **GEARY, Nicholas William**
**Cincinnati, Ohio 45202 (US)**

(74) Representative: **Siddiquee, Sanaul Kabir**
**N.V. Procter & Gamble**
**Services Company S.A.**
**Temselaan 100**
**1853 Strombeek-Bever (BE)**

(54) **CLEANING COMPOSITION**

(57) The present invention is directed to a cleaning composition comprising surface active proteins selected from the group consisting of ranaspumins, latherins, and mixtures thereof and a specific surfactant system comprising one or more anionic surfactants and one or more co-surfactants and wherein the weight ratio of the anionic surfactants to the co-surfactants is less than 9:1. Methods of making and using such compositions are also provided.

EP 3 483 248 A1

SEQUENCE LISTING

<110>  The Procter & Gamble Company

<120>  Cleaning composition

<130>  CM04898FM

<160>  14

<170>  PatentIn version 3.5

<210>  1
<211>  97
<212>  PRT
<213>  Engystomops pustulosus

<400>  1

```
Met Leu Ile Leu Asp Gly Asp Leu Leu Lys Asp Lys Leu Lys Leu Pro
1               5                   10                  15


Val Ile Asp Asn Leu Phe Gly Lys Glu Leu Leu Asp Lys Phe Gln Asp
            20                  25                  30


Asp Ile Lys Asp Lys Tyr Gly Val Asp Thr Lys Asp Leu Lys Ile Leu
        35                  40                  45


Lys Thr Ser Glu Asp Lys Arg Phe Tyr Tyr Val Ser Val Asp Ala Gly
    50                  55                  60


Asp Gly Glu Lys Cys Lys Phe Lys Ile Arg Lys Asp Val Asp Val Pro
65                  70                  75                  80


Lys Met Val Gly Arg Lys Cys Arg Lys Asp Asp Asp Asp Asp Asp Gly
                85                  90                  95


Tyr
```

<210>  2
<211>  217
<212>  PRT
<213>  Leptodactylus vastus

<400>  2

```
Leu Leu Glu Gly Phe Leu Val Gly Gly Gly Val Pro Gly Pro Gly Thr
1               5                   10                  15


Ala Cys Leu Thr Lys Ala Leu Lys Asp Ser Gly Asp Leu Leu Val Glu
            20                  25                  30


Leu Ala Val Ile Ile Cys Ala Tyr Gln Asn Gly Lys Asp Leu Gln Glu
```

35 40 45

Gln Asp Phe Lys Glu Leu Lys Glu Leu Leu Glu Arg Thr Leu Glu Arg
50 55 60

Ala Gly Cys Ala Leu Asp Asp Ile Val Ala Asp Leu Gly Leu Glu Glu
65 70 75 80

Leu Leu Gly Ser Ile Gly Val Ser Thr Gly Asp Ile Ile Gln Gly Leu
85 90 95

Tyr Lys Leu Leu Lys Glu Leu Lys Ile Asp Glu Thr Val Phe Asn Ala
100 105 110

Val Cys Asp Val Thr Lys Lys Met Leu Asp Asn Lys Cys Leu Pro Lys
115 120 125

Ile Leu Gln Gly Asp Leu Val Lys Phe Leu Lys Asp Leu Lys Tyr Lys
130 135 140

Val Cys Ile Glu Gly Gly Asp Pro Glu Leu Ile Ile Lys Asp Leu Lys
145 150 155 160

Ile Ile Leu Glu Arg Leu Pro Cys Val Leu Gly Gly Val Gly Leu Asp
165 170 175

Asp Leu Phe Lys Asn Ile Phe Val Lys Asp Gly Ile Leu Ser Phe Glu
180 185 190

Gly Ile Ala Lys Pro Leu Gly Asp Leu Leu Ile Leu Val Leu Cys Pro
195 200 205

Asn Val Lys Asn Ile Asn Val Ser Ser
210 215

<210> 3
<211> 218
<212> PRT
<213> Leptodactylus fuscus

<400> 3

Arg Ile Ser Tyr Trp Val Gly Ala Lys Pro Ile Leu Ile His Ala Leu
1 5 10 15

Lys Asn Thr Ser Ala Leu Ile Leu Gln Leu His Ala Met Ile Cys Ile
20 25 30

Tyr Gln Asn Gly Thr Gly Asn Leu Thr Asp Ala Asp Phe Gln Lys Leu

35 40 45

Asn Ile Leu Val Asn Asn Thr Leu Ser Lys Tyr Gly Cys Thr Leu Asp
50 55 60

Met Leu Met Lys Glu Leu Gly Ile Gly Ser Leu Asn Leu Gln Asp Leu
65 70 75 80

Leu Gly Gly Leu Ser Pro Ala Val Gly Asp Thr Phe Lys Ile Ile Tyr
85 90 95

Glu Leu Leu Lys Gly Val Gln Leu Glu Lys Thr Val Leu Thr Thr Leu
100 105 110

Cys Asp Ala Ile Gly Lys Leu Ile Asn Glu Gly Cys Leu Pro Asp Leu
115 120 125

Phe Gln Pro Asp Leu Val Glu Phe Phe Lys Asn Phe Lys Lys Leu Cys
130 135 140

Glu Ser Phe Asn Ser Gly Asp Leu Asp Ala Phe Thr Lys Ser Leu Glu
145 150 155 160

Lys Val Leu Glu Arg Met Lys Cys Leu Leu His Gly Leu Ser Ile Thr
165 170 175

Asp Ile Leu Asn Asn Ile Ser Ala Gln Asp Gly Lys Leu Ile Phe Ser
180 185 190

Gly Leu Ala Lys Pro Leu Gly Asp Leu Leu Thr Thr Leu Cys Pro Leu
195 200 205

Ile Gly Lys Leu Ala Pro Ala Thr Gly Asp
210 215

<210> 4
<211> 164
<212> PRT
<213> Bufo gargarizans

<400> 4

Met Lys Thr Ile Leu Leu Leu Val Leu Ala Gly Leu Ser Val Ser Tyr
1 5 10 15

Gly Phe Asn Cys Ile Gln Arg Tyr Gln Ala Ala Thr Thr Leu Gln Cys
20 25 30

Leu Thr Asn Ile Leu Glu Ile Ala Pro Asp Phe Leu Glu Lys Leu Val

35 40 45

Tyr Phe Leu Cys Asn Tyr Asp Glu Gly Met Lys Asn Asn Lys Gly Glu
50 55 60

Phe Glu Ala Met Phe Arg Asp Leu Ile Glu Ile Leu Glu Cys Ala Gly
65 70 75 80

Cys Ala Leu Asp Glu Ile Thr Gly Thr Asn Ile Ser Ile Glu Glu Leu
85 90 95

Leu Gly Asp Ile Gly Gly Ala Gly Arg Gln Ala Leu Phe Gly Ile Leu
100 105 110

Lys Ala Ala Asp Gly Leu Gln Leu Thr Gly Thr Val Ala Asp Leu Gly
115 120 125

Cys Ser Leu Leu Lys Gly Leu Gly Thr Pro Gly Gly Leu Ile Asn Leu
130 135 140

Asn Gly Val Leu Gly Gly Leu Thr Gly Gly Leu Gly Gly Ile Leu Ser
145 150 155 160

Leu Val Lys Asn

<210> 5
<211> 135
<212> PRT
<213> Engystomops pustulosus

<400> 5

Met Ala Ala Ile Gln Phe Ala Leu Phe Phe Val Phe Ala Val Ile Ser
1 5 10 15

Gln His Cys Ala Tyr Gly Phe Leu Pro Leu Gly Gly Gly Asn Ile Gly
20 25 30

Gly Gly Ala Lys Leu Gly Pro Glu Lys Pro Ala Thr Pro Gly Ile Gln
35 40 45

Asp Leu Leu Lys Ser Leu Leu Ser Val Leu Asn Leu Ser Pro Pro Ala
50 55 60

Ile Pro Glu Asp Ala Glu Ala Val Ser Tyr Arg Asp Ala Lys Asn Gly
65 70 75 80

Lys Phe Arg Leu Ile Lys Ile His Leu Gly Gly Glu Leu Tyr Cys His

85 90 95

Val Lys Gln Ile Ala Gly Pro Ile Leu Ala Leu Pro Ile Val Ser Asp
100 105 110

Val Val Glu Val Thr Gly Lys Glu Cys Gly Lys Thr Glu Asp Asp Pro
115 120 125

Leu Glu Asp Phe Pro Ile Pro
130 135

<210> 6
<211> 174
<212> PRT
<213> Engystomops pustulosus

<400> 6

Met Ile Asp Pro Thr Gly Leu Val Gln Ile Leu Leu Leu Glu Gln Val
1 5 10 15

Val His Lys Ile Pro Pro Gly Asn Ile Asn Leu Ala Arg Thr Gly Ile
20 25 30

Ala Thr Gln Asp Ser Asp Tyr Thr Ala Ser Ala Val Pro Ser Glu Ala
35 40 45

Arg Leu Ala Ile Asp Gly Asn Arg Asn Ser Asp Phe Asn Gln Lys Ser
50 55 60

Cys Ser His Thr Gly Gly Asn Glu Pro Ala Trp Trp Arg Leu Glu Leu
65 70 75 80

Lys Lys Lys Ser Lys Ile Ser Val Val Val Ile Ala Ile Arg Ser Asp
85 90 95

Cys Cys Met Asp Arg Phe Lys Gly Ala Glu Leu Arg Ile Gly Asn Ser
100 105 110

Gln Asp Ala Thr Val Asn Pro Ile Cys Gly Lys Val Ser Ala Val Lys
115 120 125

Gly Ser Asn Tyr Leu Phe Cys Cys Asp Gly Met Glu Gly Lys Tyr Ile
130 135 140

Ser Val Val Ile Pro Asp Arg His Glu Phe Leu Ser Leu Cys Glu Val
145 150 155 160

Glu Val Tyr Gly Ala Lys Pro Ile Glu Gly Thr His Cys Lys

165 170

<210> 7
<211> 193
<212> PRT
<213> Engystomops pustulosus

<400> 7

Met Lys Leu Leu Leu Leu Val Val Leu Val Trp Thr Ala Ser Asp Glu
1 5 10 15

Ala Ser Ala Asp Arg Asn Leu Ala Leu Asp Gly Arg Ala Thr Met Ser
20 25 30

Ser Ile Trp Met Asp Pro Asp Ile Arg Gln Ser Phe Leu Gly Val Ala
35 40 45

Met Asn Gly Ile Asp Gly Asn Thr Asp Ser Val Tyr Phe His Gly Ser
50 55 60

Cys Phe His Thr Gly Leu Asp Ser Pro Ala Trp Tyr Arg Val Asp Leu
65 70 75 80

Leu Arg Thr Ser Lys Ile Ser Ser Ile Thr Ile Thr Asn Arg Gly Asp
85 90 95

Phe Gly Ser Arg Thr Asn Gly Ala Glu Ile Arg Ile Gly Asp Ser Leu
100 105 110

Ala Asn Asn Gly Asn Asn Asn Pro Arg Cys Ala Leu Val Thr Ser Ile
115 120 125

Ala Asp Gly Glu Thr Arg Thr Phe Gln Cys Asn Asn Met Val Gly Arg
130 135 140

Tyr Val Asn Ile Val Leu Thr Gly Lys Thr Glu Phe Leu His Leu Cys
145 150 155 160

Glu Val Gln Ile Phe Gly Glu Asn Leu Pro Arg Ser Phe Ser Cys Gln
165 170 175

Tyr Ser Asn Asp Gly Met Ile Thr Leu Leu Val Ser Thr Arg Phe Met
180 185 190

Lys

<210> 8

<211> 178
<212> PRT
<213> Engystomops pustulosus

<400> 8

Met Gly Ala Pro Gly Gly Ala Ala Gly Pro Leu Leu Val Leu Asn Ile
1 5 10 15

Leu Gly Ser Val Val His Glu Thr Lys Pro Pro Glu Gly Val Asn Leu
20 25 30

Ala Leu Lys Gly Ile Ala Ser Ser Asp Ser Ile Ala Ser Asn Gly Ser
35 40 45

Val Thr Gly Leu Ala Ala Lys Ala Ile Asp Gly Ile Arg Val Ser Asp
50 55 60

Phe Phe Lys Gly His Cys Ser Leu Thr Asn Gly Leu Asn Asn Pro Thr
65 70 75 80

Trp Trp Lys Val Asp Leu Lys Lys Ser Tyr Lys Ile Ser Ser Val Phe
85 90 95

Val Thr Asn Arg Asp Asp Cys Cys Thr Glu Arg Leu Leu His Ala Glu
100 105 110

Ile Arg Ile Gly Ser Asn Pro Asp His Asn His Asn Pro Ile Cys Ala
115 120 125

Glu Val Lys Thr Val Ala Ser Ser Asn Ile Gly Phe Cys Cys Gly Gly
130 135 140

Met Glu Gly Arg Tyr Val Ser Val Ser Val Pro Arg Lys Glu Gln Leu
145 150 155 160

Ser Leu Cys Glu Val Glu Val Tyr Gly Asp Leu Lys Lys Val Leu His
165 170 175

Cys Ala

<210> 9
<211> 246
<212> PRT
<213> Engystomops pustulosus

<400> 9

Met Ile Leu Ile Leu Gly Val Leu Leu Leu Gly Ala Glu Ala Ser Ala
1 5 10 15

```
Glu Thr Leu Cys Ile Pro Gly Arg Met Lys Gln Leu Asp Ala Gly Ala
            20                  25                  30

Gly Arg Val Val Ala Val Lys Ser Asn Gly Asp Val Tyr Gln Leu Leu
        35                  40                  45

Glu Asn Asn Trp Val Gln Ile Pro Gly Lys Leu Ile His Val Thr Val
    50                  55                  60

Gly Pro Ala Gly Leu Trp Gly Val Asn Lys Asp Lys Asn Ile Tyr Lys
65                  70                  75                  80

Tyr Val Asp Asn Asp Trp Leu Gln Val Asp Gly Leu Leu Asn Gln Ile
                85                  90                  95

Asp Ala Gly Gly Asn Arg Phe Val Val Gly Val Asn Asp Asn Glu Asp
            100                 105                 110

Ile Phe Cys Leu Asn Gln Asp Gln Thr Thr Ser Asn Ala Val Lys Leu
        115                 120                 125

Asp Tyr Lys Gly Val Asp Gly Lys Leu Lys Tyr Tyr Ser Ser Gly Gly
    130                 135                 140

Tyr Gly Ser Trp Gly Val Asn Ala Ala Tyr Asp Ile Phe Tyr Arg Arg
145                 150                 155                 160

Asn Val His Pro Met Ser Cys Gln Gly Thr Asn Trp Glu Asn Val Glu
                165                 170                 175

Gly Lys Leu Val Met Leu Glu Val Ala Glu Asp Gly Ser Val Tyr Gly
            180                 185                 190

Val Asn Tyr Asn Gly His Val Tyr Lys Arg Glu Gly Ile Thr Ala Gly
        195                 200                 205

Asn Pro Met Gly Thr Ser Trp Thr Tyr Leu Lys Val Asp Glu Lys Val
    210                 215                 220

Arg His Val Ser Tyr Asp Arg Gly Val Leu Tyr Val Val Thr Ile Asp
225                 230                 235                 240

Asp Arg Ile Phe Arg Cys
                245
```

<210> 10

<211> 228
<212> PRT
<213> Equus caballus

<400> 10

```
Met Leu Lys Val Ser Cys Leu Phe Val Leu Leu Cys Gly Leu Leu Val
1               5                   10                  15

Pro Ser Ser Ala Gln Gln Ile Pro Pro Glu Val Ser Ser Gln Ile Thr
            20                  25                  30

Asp Ala Leu Thr Gln Gly Leu Leu Asp Gly Asn Phe Leu Ser Leu Leu
        35                  40                  45

Asn Ala Ile Asn Leu Glu Gly Leu Leu Asn Thr Ile Leu Asp Gln Val
    50                  55                  60

Thr Gly Leu Leu Asn Ile Leu Val Gly Pro Leu Leu Gly Pro Ser Asp
65                  70                  75                  80

Ala Glu Ile Lys Leu Gln Asp Thr Arg Leu Leu Gln Leu Ser Leu Glu
                85                  90                  95

Phe Ser Pro Asp Ser Lys Gly Ile Asp Ile Trp Ile Pro Leu Glu Leu
            100                 105                 110

Ser Val Tyr Leu Lys Leu Leu Ile Leu Glu Pro Leu Thr Leu Tyr Val
        115                 120                 125

Arg Thr Asp Ile Arg Val Gln Leu Arg Leu Glu Ser Asp Glu Asp Gly
    130                 135                 140

Lys Tyr Arg Leu Ala Phe Gly His Cys Ser Leu Leu Pro Arg Ala Ile
145                 150                 155                 160

Glu Leu Gln Ser Gly Asn Pro Leu Ser Leu Pro Val Asn Ala Val Leu
                165                 170                 175

Gly Thr Ile Glu Asn Ala Leu Gly Asn Phe Ile Thr Glu Asp Leu Gly
            180                 185                 190

Ala Gly Leu Cys Pro Thr Leu Asn Ser Leu Val Ser Asn Leu Asp Leu
        195                 200                 205

Gln Leu Val Asn Asn Leu Ile Asn Leu Ile Leu Asp Arg Ala Asn Val
    210                 215                 220

Asp Leu Ser Val
```

225

<210> 11
<211> 290
<212> DNA
<213> None

<400> 11
atgctgatcc tggacggtga tctgctgaag gacaaactga agctgccggt gattgataac 60

ctgttcggta agagctgctg gacaagtttc aggacgatat caaggataag tacggcgttg 120

acaccaagga tctgaaaatt ctgaagacca gcgaggacaa acgtttctac tatgtgagcg 180

ttgacgcggg tgatggcgaa aaatgcaagt ttaaaatccg taaagacgtg gatgttccga 240

agatggtggg tcgtaaatgc cgtaaggacg atgacgatga cgatggctat 290

<210> 12
<211> 111
<212> PRT
<213> Engystomops pustulosus

<400> 12

Met His His His His His His Glu Asn Leu Tyr Phe Gln Gly Met Leu
1 5 10 15

Ile Leu Asp Gly Asp Leu Leu Lys Asp Lys Leu Lys Leu Pro Val Ile
20 25 30

Asp Asn Leu Phe Gly Lys Glu Leu Leu Asp Lys Phe Gln Asp Asp Ile
35 40 45

Lys Asp Lys Tyr Gly Val Asp Thr Lys Asp Leu Lys Ile Leu Lys Thr
50 55 60

Ser Glu Asp Lys Arg Phe Tyr Tyr Val Ser Val Asp Ala Gly Asp Gly
65 70 75 80

Glu Lys Cys Lys Phe Lys Ile Arg Lys Asp Val Asp Val Pro Lys Met
85 90 95

Val Gly Arg Lys Cys Arg Lys Asp Asp Asp Asp Asp Asp Gly Tyr
100 105 110

<210> 13
<211> 630
<212> DNA
<213> NOne

<400> 13
ttgttagaag gtttcttggt tggtggtggt gttccaggtc caggtactgc ttgtttgaca 60

```
aaagcattaa aagattctgg tgacttgttg gttgaattgg ctgttattat ctgtgcatac      120

caaaacggta aagatttgca agaacaagat ttcaaggaat tgaaggaatt gttggaaaga      180

actttagaaa gagctggttg tgcattggat gatattgttg ctgatttggg tttagaagaa      240

ttgttaggtt ctattggtgt ttcaacaggt gacattattc aaggtttgta caagttgttg      300

aaggaattga agattgatga aactgttttt aatgcagttt gtgatgttac taagaaaatg      360

ttggataata agtgtttgcc aaagatcttg cagggtgact tggttaagtt cttgaaggat      420

ttgaagtaca aggtttgtat tgaaggtggt gacccagaat tgattattaa ggatttgaag      480

atcatcttgg aaagattacc atgtgttttg ggtggtgttg gtttggatga tttgtttaaa      540

aacatcttcg ttaaggatgg tatcttgtca ttcgaaggta tcgctaaacc attgggtgac      600

ttgttaattt tggttttatg tccaaatgtt                                        630
```

```
<210>  14
<211>  230
<212>  PRT
<213>  Leptodactylus vastus

<400>  14

His His His His His His Glu Asn Leu Tyr Phe Gln Gly Leu Leu Glu
1               5                   10                  15

Gly Phe Leu Val Gly Gly Gly Val Pro Gly Pro Gly Thr Ala Cys Leu
            20                  25                  30

Thr Lys Ala Leu Lys Asp Ser Gly Asp Leu Leu Val Glu Leu Ala Val
        35                  40                  45

Ile Ile Cys Ala Tyr Gln Asn Gly Lys Asp Leu Gln Glu Gln Asp Phe
    50                  55                  60

Lys Glu Leu Lys Glu Leu Leu Glu Arg Thr Leu Glu Arg Ala Gly Cys
65                  70                  75                  80

Ala Leu Asp Asp Ile Val Ala Asp Leu Gly Leu Glu Glu Leu Leu Gly
                85                  90                  95

Ser Ile Gly Val Ser Thr Gly Asp Ile Ile Gln Gly Leu Tyr Lys Leu
            100                 105                 110

Leu Lys Glu Leu Lys Ile Asp Glu Thr Val Phe Asn Ala Val Cys Asp
        115                 120                 125

Val Thr Lys Lys Met Leu Asp Asn Lys Cys Leu Pro Lys Ile Leu Gln
    130                 135                 140
```

```
Gly Asp Leu Val Lys Phe Leu Lys Asp Leu Lys Tyr Lys Val Cys Ile
145                 150                 155                 160

Glu Gly Gly Asp Pro Glu Leu Ile Ile Lys Asp Leu Lys Ile Ile Leu
                165                 170                 175

Glu Arg Leu Pro Cys Val Leu Gly Gly Val Gly Leu Asp Asp Leu Phe
            180                 185                 190

Lys Asn Ile Phe Val Lys Asp Gly Ile Leu Ser Phe Glu Gly Ile Ala
        195                 200                 205

Lys Pro Leu Gly Asp Leu Leu Ile Leu Val Leu Cys Pro Asn Val Lys
    210                 215                 220

Asn Ile Asn Val Ser Ser
225                 230
```

## Description

REFERENCE TO A SEQUENCE LISTING

This application contains Sequence Listings in computer readable form. The computer readable form is incorporated herein by reference.

FIELD OF THE INVENTION

The present invention relates to a cleaning composition comprising a surface active protein and a specific surfactant system. The composition provides one or more benefits, including good cleaning particularly good grease emulsification, long lasting suds especially in presence of greasy soils and surface modification that can contribute to second time cleaning benefits, improved drying, improved shine in the case of dishware, etc.

BACKGROUND OF THE INVENTION

Cleaning compositions should provide good soil and/or grease cleaning while presenting a good suds profile in particular a long lasting suds profile especially in the presence of greasy soils. Users usually see suds as an indicator of the performance of the cleaning composition. Moreover, the user of a cleaning composition may also use the suds profile and the appearance of the suds (e.g., density, whiteness) as an indicator that the wash solution still contains active cleaning ingredients. This is particularly the case for manual washing, also referred to herein as hand-washing, where the user usually doses the cleaning composition depending on the suds remaining and renews the wash solution when the suds subsides or when the suds does not look thick enough. Thus, a cleaning composition, particularly a manual wash cleaning composition that generates little or low density suds would tend to be replaced by the user more frequently than is necessary. Accordingly, it is desirable for a cleaning composition to provide "good sudsing profile", which includes good suds height and/or density as well as good suds duration during the initial mixing of the composition with water and/or during the entire washing operation.

Several families of natural surface active proteins are able to aid suds performance in aqueous solutions (*see* Cooper, A., et al. (2017), Colloids Surf., A: Physiochemical and Engineering Aspects*;* Schor, M., et al. (2016), Trends Biochem. Sci. 41(7): 610-620). For example, ranaspumins produced by different frog species and latherin from equines have been previously described in the art (Fleming, R. I., et al. (2009), Proc. R. Soc. B 276(1663): 1787-1795, and Beeley, J. G., et al. (1986), Biochem. J. 235(3): 645-650). However, the amount of sudsing generated by such surface active proteins in cleaning formulations is limited.

Accordingly, the need remains for an improved cleaning composition comprising one or more surface active proteins which have a further improved sudsing profile, particularly at low surface active proteins concentrations in the cleaning composition. The need also exists for an improved cleaning composition, when used in a manual-washing process, the composition should also provide a pleasant washing experience, *i.e.,* good feel on the user's hands during the wash. The composition should also be easy to rinse. Further it is desirous that the improved cleaning composition is stable and will not phase separate, resulting in greater shelf-life of the product. It is also desirable that cleaning compositions provide surface modification, contributing to shine in the case of dishware, improved second time cleaning. There is also the desire to reduce the amount of surfactants without negatively impacting sudsing nor grease cleaning and emulsification profile. Thus, there is the need to find new compositions that improve cleaning, suds longevity and improved after cleaning benefits in hand washing conditions. The Applicant discovered that some or all of the above-mentioned needs can be at least partially fulfilled through the improved cleaning composition as described herein below.

SUMMARY OF THE INVENTION

The present invention meets one or more of these needs based on the surprising discovery that by formulating a detergent composition comprising a surface active protein and a specific surfactant system, such a composition exhibits good sudsing profile, particularly desirable suds volume and/or sustained suds stabilization, especially in the presence of greasy soils. It also provides good grease cleaning and emulsification benefits and can also provide surface modifications facilitating next time cleaning benefit.

According to the present invention there is provided a cleaning composition comprising one or more surface active proteins selected from the group consisting of ranaspumins, latherins, and mixtures thereof, preferably ranaspumins, and a surfactant system.

Preferably the cleaning composition is a manual-washing composition. Preferably the cleaning composition is for manual dishwashing. Preferred compositions are in the form of a liquid.

The composition of the invention provides good cleaning and good suds profile, especially in the presence of

greasy soils. It can also provide surface modifications facilitating next time cleaning benefit.

According to the present invention, there is provided a method of manual washing comprising the steps of: a) delivering the cleaning composition of the invention to a volume of water to form a wash liquor; and b) immersing the soiled articles into said wash liquor. When the composition of the invention is used according to this method a good sudsing profile, with a long lasting effect is achieved.

In another aspect, the present invention is directed to a method of manually washing dishware comprising the steps of delivering a detergent composition of the invention into a volume of water to form a wash solution and immersing the dishware in the solution.

In yet another aspect, the present invention relates to a method of manually washing dishware comprising: i) delivering a detergent composition of the present invention onto the dishware or a cleaning implement; ii) cleaning the dishware with the composition in the presence of water; and iii) optionally, rinsing the dishware. Preferably, the composition of the present invention is used in neat form (*i.e.,* direct application) since greater benefits in terms of grease cleaning are obtained when the composition is directly applied on the soiled surface or on a cleaning implement, such as a sponge, to be used to clean the soiled surface.

There is also provided the use of surface active proteins to provide increased suds longevity, increased grease emulsification or both in an aqueous wash liquor during a washing process, preferably a manual dishwashing process, preferably the surface active proteins are selected from the group consisting of ranaspumins, latherins, and mixtures thereof, preferably ranaspumins.

Preferably the manual washing is dishwashing and the soiled articles comprise soiled dishware. As used herein, "dishware" includes cookware and tableware.

The elements of the composition of the invention described in relation to the first aspect of the invention apply *mutatis mutandis* to the other aspects of the invention.

These and other features, aspects and advantages of the present invention will become evident to those skilled in the art from the detailed description which follows.

## DETAILED DESCRIPTION OF THE INVENTION

### Definitions

As used herein, the articles "a" and "an" when used in a claim, are understood to mean one or more of what is claimed or described.

As used herein, the term "substantially free of' or "substantially free from" means that the indicated material is present in an amount of no more than about 5 wt%, preferably no more than about 2%, and more preferably no more than about 1 wt% by weight of the composition.

As used therein, the term "essentially free of' or "essentially free from" means that the indicated material is present in an amount of no more than about 0.1 wt% by weight of the composition, or preferably not present at an analytically detectible level in such composition. It may include compositions in which the indicated material is present only as an impurity of one or more of the materials deliberately added to such compositions.

As used herein, the term "amino acid identity" means the identity between two or more amino acid sequences and is expressed in terms of the identity or similarity between the sequences. Sequence identity can be measured in terms of percentage identity; the higher the percentage, the more identical the sequences are. The percentage identity is calculated over the length of comparison. For example, the amino acid identity is typically calculated over the entire length of a sequence aligned against the entire length of the reference sequence (e.g., SEQ ID NO: 6, SEQ ID NO: 7, etc.). Methods of alignment of sequences for comparison are well known in the art and identity can be calculated by many known methods. Various programs and alignment algorithms are described in the art. It should be noted that the terms 'sequence identity' and 'sequence similarity' can be used interchangeably.

As used herein, the term "surface active proteins" refer to the wild-type surface proteins selected from the group consisting of ranaspumins, latherins, and mixtures thereof, and variants thereof.

As used herein, the term "cleaning composition" refers to a composition or formulation designed for cleaning soiled surfaces. Such compositions include but are not limited to, dishwashing compositions, laundry detergent compositions, fabric softening compositions, fabric enhancing compositions, fabric freshening compositions, laundry pre-wash, laundry pretreat, laundry additives, spray products, dry cleaning agent or composition, laundry rinse additive, wash additive, post-rinse fabric treatment, ironing aid, hard surface cleaning compositions, unit dose formulation, delayed delivery formulation, detergent contained on or in a porous substrate or nonwoven sheet, and other suitable forms that may be apparent to one skilled in the art in view of the teachings herein. Such compositions may be used as a pre-cleaning treatment, a post-cleaning treatment, or may be added during the rinse or wash cycle of the cleaning process. The cleaning compositions may have a form selected from liquid, powder, single-phase or multiphase unit dose or pouch form, tablet, gel, paste, bar, or flake. Preferably the composition is for manual-washing. Preferably, the cleaning composition of the present invention is a dishwashing detergent. Preferably the composition is in the form of a liquid.

As used herein the term "fragment" means an amino acid sequence of at least 20, 40, 60, 80, 100, 150 contiguous amino acids of the reference sequences or any integer there between.

As used herein the term "improved suds longevity" means an increase in the duration of visible suds in a washing process cleaning soiled articles using the composition comprising one or more surface active proteins, compared with the suds longevity provided by the same composition and process in the absence of the surface active proteins.

As used herein, the term "next time cleaning benefit" means the surface to be cleaned could be treated with a composition which would assist in easier removal of soil and/or stains during subsequent cleaning.

As used herein, the term "soiled surfaces" refers non-specifically to any type of flexible material consisting of a network of natural or artificial fibers, including natural, artificial, and synthetic fibers, such as, but not limited to, cotton, linen, wool, polyester, nylon, silk, acrylic, and the like, as well as various blends and combinations. Soiled surfaces may further refer to any type of hard surface, including natural, artificial, or synthetic surfaces, such as, but not limited to, tile, granite, grout, glass, composite, vinyl, hardwood, metal, cooking surfaces, plastic, and the like, as well as blends and combinations, as well as dishware. Key targeted soiled surfaces by this application are soiled dishware.

As used herein, the term "variant" of the surface active proteins means an amino acid sequence when the surface active protein is modified by, or at, one or more amino acids (for example 1, 2, 3, 4, 5, 6, 7, 8, 9 or 10 or more amino acid modifications) selected from substitutions, insertions, deletions and combinations thereof. The variant may have "conservative" substitutions, wherein a substituted amino acid has similar structural or chemical properties to the amino acid that replaces it, for example, replacement of leucine with isoleucine. A variant may have "non-conservative" changes, for example, replacement of a glycine with a tryptophan. Variants may also include sequences with amino acid deletions or insertions, or both. Guidance in determining which amino acid residues may be substituted, inserted, or deleted without abolishing the activity of the protein may be found using computer programs well known in the art. Variants may also include truncated forms derived from a wild-type surface active protein, such as for example, a protein with a truncated N-terminus or C-terminus. Variants may also include forms derived by adding an extra amino acid sequence to a wild-type protein, such as for example, an N-terminal tag, a C-terminal tag or an insertion in the middle of the protein sequence.

As used herein, the term "water hardness" or "hardness" means uncomplexed cation ions (*i.e.,* $Ca^{2+}$ or $Mg^{2+}$) present in water that have the potential to precipitate with anionic surfactants or other anionic actives in the cleaning composition under alkaline conditions, and thereby diminishing the surfactancy and cleaning capacity of surfactants. Further, the terms "high water hardness" and "elevated water hardness" can be used interchangeably and are relative terms for the purposes of the present invention, and are intended to include, but not limited to, a hardness level containing at least 12 grams of calcium ion per gallon water (gpg, "American grain hardness" units).

## Cleaning Composition

A preferred cleaning composition is a manual dishwashing composition, preferably in liquid form. It typically contains from 30% to 95%, preferably from 40% to 90%, more preferably from 50% to 85% by weight of the composition of a liquid carrier in which the other essential and optional components are dissolved, dispersed or suspended. One preferred component of the liquid carrier is water.

Preferably the pH of the cleaning composition of the invention, measured as a 10% product concentration in demineralized water at 20°C, is adjusted to between 3 and 14, more preferably between 4 and 13, more preferably between 6 and 12 and most preferably between 8 and 10. The pH of the cleaning composition can be adjusted using pH modifying ingredients known in the art.

## Surface Active Proteins

Ranaspumins (from Latin: *rana* (frog) and *spuma* (foam)) are proteins originally characterized from the suds nest material produced by the tungara frog (*Engystomops pustulosus*). In this particular specie, six main ranaspumins (designated as Ep-Rsn1, Ep-Rsn2, Ep-Rsn3, Ep-Rsn4, Ep-Rsn5, and Ep-Rsn6) with different biological roles related to suds formation and stability have been identified. From these proteins, Ep-Rsn2 (SEQ ID NO: 1) is the major surface active protein in the suds mixture, while the other ranaspumins contribute mostly to suds stability. Ep-Rsn2 has no homology to any other protein or domains presently reported in the protein sequences databases. Interestingly, the Ep-Rsn2 sequence shows an unusual distribution of amino acid residues, including a highly hydrophobic N-terminal region (LILDGDLLK-) and a highly charged C-terminal region (-RKDDDDDDGY), suggesting its potential role as a surface activity protein. Structural analysis revealed that Ep-Rsn2 comprises a four-stranded antiparallel β sheet with an α helix lying across one side of the sheet, similar to cystatins. The flexible N-terminal unstructured tail is expected to capture hydrophobic interfaces, followed by a large conformational change where the helix moves apart from the sheet revealing the hydrophobic core of the protein.

Protein Ep-Rsn1 (SEQ ID NO: 5) has some amino acid sequence similarity to cystatins (cysteinyl proteinase inhibitors), but does not appear to have similar inhibitory activity. Instead, Ep-Rsn1 reduces aqueous surface tension, though not at the same level than Ep-Rsn1. Proteins Ep-Rsn3 (SEQ ID NO: 6), Ep-Rsn4 (SEQ ID NO: 7), and Ep-Rsn5 (SEQ ID NO: 8) are similar to each other and have some sequence similarity to a family of fucose-binding proteins, also known as "fucolectins", whereas Ep-Rsn6 (SEQ ID NO: 9) belongs to a different type of lectins (C-type) frequently associated with galactose binding. The carbohydrate-binding activity of Ep-Rsn4 has been confirmed experimentally. Furthermore, Ep-Rsn3 and Ep-Rsn5 have hydrophobic N-terminal tails that might serve to anchor them at the interface.

The role of Ep-Rsn3, Ep-Rsn4, Ep-Rsn5, and Ep-Rsn6 in suds stabilization has been suggested in the art. It is believed that initial suds formation is facilitated by Ep-Rsn2 (and possibly Ep-Rsnl), while the rest of the ranaspumins build a more complex layer, possibly by binding to long-chain branched polysaccharide molecules, creating a mechanically stable interface. Indeed, the suds from *E. pustulosus* contain not only proteins, but also significant amounts of carbohydrates, predominantly complex cross-linked mixtures of O- and N-glycans.

Composition analysis of the suds nests of *Leptodactylus vastus,* an unrelated frog species, allowed the identification of a mixture of proteins, including the surface active protein Lv-Rsnl (SEQ ID NO: 2). This protein is much bigger than Ep-Rsn2 and comprises two domains and four disulfide bridges that stabilize the structure. It is believed that Lv-Rsnl undergoes a conformational change to facilitate interfacial association. Despite similar functions, Lv-Rsnl is totally unrelated to Ep-Rsn2, but has homology to proteins produced by *Leptodactylus fuscus,* designed as Lf-Rsn1 (SEQ ID NO: 3), and from *Bufo gargarizans,* designated as Bg-Rsn1 (SEQ ID NO: 4).

Latherins are proteins found in sweat and saliva of horses and other equines. One of the biological roles of latherins is enabling wetting of the oily, waterproof hairs, aiding fast flow of sweat from the glands, through the thick pelts, to the air interface. The amino acid sequences of latherin from different equine species are highly conserved. They belong to the group of PLUNC (palate, lung, and nasal epithelium clone) proteins expressed in mammalian salivary glands and oral cavities.

The amino acid sequence of *Equus caballus* latherin (SEQ ID NO: 10) is characterized by an unusually high leucine content (about 24%), which may be related to its surface properties. However, the solution structure of latherin does not display any major hydrophobic regions, suggesting that conformational changes might be required for interfacial association of the protein.

Unexpectedly, the Applicants found that one or more surface active proteins, in particular, surface active proteins selected from the group consisting of ranaspumins, latherins, and mixtures thereof, preferably ranaspumins, in combination with a specific surfactant system, is able to produce a more stable hence longer lasting sudsing profile in detergent wash solutions comprising oily and/or greasy soils. Not wishing to be bound by theory, the Applicants believe that the increased sudsing benefits are due to conformational changes of the proteins that expose hydrophobic patches and generate amphiphilic structures that can associate and stabilize interfaces (*i.e.,* oil-water interface or air-water interface).

Accordingly, the cleaning composition in accordance with the present invention comprises one or more surface active proteins selected from the group consisting of ranaspumins, latherins, and mixtures thereof, preferably ranaspumins.

The ranaspumins have at least 90%, preferably at least 95%, preferably at least 98% or even 100% amino acid identity as calculated over the entire length of the sequence aligned against the entire length of at least one reference sequence selected the group consisting of: *Engystomops pustulosus* Ep-Rsn2 (SEQ ID NO: 1), *Leptodactylus vastus* Lv-Rsnl (SEQ ID NO: *2), Leptodactylus fuscus* Lf-Rsn1 (SEQ ID NO: 3), and *Bufo gargarizans* Bg-Rsn (SEQ ID NO: 4), more preferably *Engystomops pustulosus* Ep-Rsn2 (SEQ ID NO: 1) and *Leptodactylus vastus* Lv-Rsnl (SEQ ID NO: 2).

The latherins have at least 90%, preferably at least 95%, preferably at least 98% or even 100% amino acid identity as calculated over the entire length of the sequence aligned against the entire length of *Equus caballus* latherin (SEQ ID NO: 10).

Preferably the cleaning composition further comprises one or more co-proteins selected from the group of lactins. Non-limiting examples of lactins are *Engystomops pustulosus* Ep-Rsn3 (SEQ ID NO: 6), *Engystomops pustulosus* Ep-Rsn4 (SEQ ID NO: 7), *Engystomops pustulosus* Ep-Rsn5 (SEQ ID NO: 8), and *Engystomops pustulosus* Ep-Rsn6 (SEQ ID NO: 9).

Preferably the cleaning composition further comprises one or more co-proteins wherein the co-proteins have at least 40%, preferably at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of at least one reference sequence selected from the group consisting of: *Engystomops pustulosus* Ep-Rsn1 (SEQ ID NO: 5), *Engystomops pustulosus* Ep-Rsn3 (SEQ ID NO: 6), *Engystomops pustulosus* Ep-Rsn4 (SEQ ID NO: 7), *Engystomops pustulosus* Ep-Rsn5 (SEQ ID NO: 8), and *Engystomops pustulosus* Ep-Rsn6 (SEQ ID NO: 9); and mixtures thereof, preferably *Engystomops pustulosus* Ep-Rsn3 (SEQ ID NO: 6) and *Engystomops pustulosus* Ep-Rsn5 (SEQ ID NO: 8).

The present invention also includes variants of ranaspumins and latherins. Variants of ranaspumins or latherins, as used herein, include a sequence resulting when a wild-type protein of the respective protein is modified by, or at, one

or more amino acids (for example 1, 2, 5 or 10 amino acids). The invention also includes variants in the form of truncated forms derived from a wild-type ranaspumin or wild type latherin, such as a protein with a truncated N-terminus or a truncated C-terminus. Some ranaspumins (*e.g.*, Ep-Rsn1, Ep-Rsn4, and Ep-Rsn5) and latherin (SEQ ID NO: 10) are predicted to include an N-terminal signal peptide that is likely removed upon secretion by the cell. The present invention includes variants without the N-terminal signal peptide. Bioinformatic tools, such as SignalP ver 4.1 (Petersen TN., Brunak S., von Heijne G. and Nielsen H. (2011), Nature Methods, 8:785-786), can be used to predict the existence and length of such signal peptides. The invention also includes variants derived by adding an extra amino acid sequence to a wild-type protein, such as for example, an N-terminal tag, a C-terminal tag or an insertion in the middle of the protein sequence. Non-limiting examples of tags are maltose binding protein (MBP) tag, glutathione S-transferase (GST) tag, thioredoxin (Trx) tag, His-tag, and any other tags known by those skilled in art. Tags can be used to improve solubility and expression levels during fermentation or as a handle for enzyme purification. For example, His6-Ep-Rns2 (SEQ ID NO: 12) is a variant of Ep-Rns2 (SEQ ID NO: 1) including an N-terminal His tag and His6-Lv-Rns1 (SEQ ID NO: 14) is a variant of Lv-Rns1 (SEQ ID NO: 2) also including the same tag.

It is important that variants of ranaspumins and latherins retain and preferably improve the ability of the wild-type protein to adsorb at an interface and to stabilize that interface. Some performance drop in a given property of variants may of course be tolerated, but the variants should retain and preferably improve suitable properties for the relevant application for which they are intended. Screening of variants of one of the wild-types can be used to identify whether they retain and preferably improve appropriate properties.

The variants may have "conservative" substitutions. Suitable examples of conservative substitution includes one conservative substitution in the peptide, such as a conservative substitution in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, or SEQ ID NO: 10. Other suitable examples include 10 or fewer conservative substitutions in the peptide, such as five or fewer. A peptide or protein of the invention may therefore include 1, 2, 3, 4, 5, 6, 7, 8, 9, 10 or more conservative substitutions. A peptide can be produced to contain one or more conservative substitutions by manipulating the nucleotide sequence that encodes that peptide using, for example, standard procedures such as site-directed mutagenesis or PCR. Alternatively, a peptide can be produced to contain one or more conservative substitutions by using peptide synthesis methods, for example, as known in the art.

Examples of amino acids which may be substituted for an original amino acid in a protein and which are regarded as conservative substitutions include: Ser for Ala; Lys for Arg; Gln or His for Asn; Glu for Asp; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

A variant includes a "modified protein" which encompasses proteins having at least one substitution, insertion, and/or deletion of an amino acid. A modified protein may have 1, 2, 3, 4, 5, 6, 7, 8, 9 or 10 or more amino acid modifications (selected from substitutions, insertions, deletions and combinations thereof).

The invention also covers any fragment of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, or SEQ ID NO: 10 that can adsorb to an interface and stabilize that interface. According to the invention, the term "fragment" is intended to mean an amino acid sequence of at least 20, 40, 60, 80 contiguous amino acids of the reference sequences or any integer there between.

Peptides can be modified by a variety of chemical techniques to produce derivatives having essentially the same or preferably even improved activity as the unmodified peptides, and optionally having other desirable properties. For example, carboxylic acid groups of the protein, whether carboxyl-terminal or side chain, may be provided in the form of a salt of a pharmaceutically-acceptable cation or esterified, for example to form a C1-C6 alkyl ester, or converted to an amide, for example of formula $CONR_1R_2$ wherein $R_1$ and $R_2$ are each independently H or C1-C6 alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C1-C6 alkyl or dialkyl amino or further converted to an amide. Hydroxyl groups of the peptide side chains may be converted to alkoxy or ester groups, for example C1-C6 alkoxy or C1-C6 alkyl ester, using well-recognized techniques. Phenyl and phenolic rings of the peptide side chains maybe substituted with one or more halogen atoms, such as F, Cl, Br or I, or with C1-C6 alkyl, C1-C6 alkoxy, carboxylic acids and esters thereof, or amides of such carboxylic acids. Methylene groups of the peptide side chains can be extended to homologous C2-C4 alkylenes. Thiols can be protected with any one of a number of well-recognized protecting groups, such as acetamide groups. Those skilled in the art will also recognize methods for introducing cyclic structures into the peptides of this disclosure to select and provide conformational constraints to the structure that result in enhanced stability.

Identity, or homology, percentages as mentioned herein in respect of the present invention are those that can be calculated with the GAP program, obtainable from GCG (Genetics Computer Group Inc., Madison, WI, USA). Alternatively, a manual alignment can be performed.

For polypeptide sequence comparison the following settings can be used: Alignment algorithm: Needleman

and Wunsch, J. Mol. Biol. 1970, 48: 443-453. As a comparison matrix for amino acid similarity the Blosum62 matrix is used (Henikoff S. and Henikoff J.G., P.N.A.S. USA 1992, 89: 10915-10919). The following gap scoring parameters are used: Gap penalty: 12, gap length penalty: 2, no penalty for end gaps.

A given sequence is typically compared against the full-length sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, or SEQ ID NO: 10 to obtain a score.

Preferably, the surface active proteins are present in an amount from 0.0001 wt% to 5 wt%, preferably from 0.01 wt% to 1 wt%, by weight of the cleaning composition, based on active protein, wherein the surface active protein is selected from selected from the group consisting of ranaspumins, latherins, and mixtures thereof, preferably ranaspumins. Preferably the ranaspumins have at least 40%, preferably at least 50%, preferably at least 60%, preferably at least 70%, preferably at least 80%, preferably at least 85%, preferably at least 90%, preferably at least 95%, preferably at least 98% or even 100% amino acid identity to at least one wild-type protein selected from the group consisting of: Engystomops pustulosus Ep-Rsn2 (SEQ ID NO: 1), Leptodactylus vastus Lv-Rsnl (SEQ ID NO: 2), Leptodactylus fuscus Lf-Rsn1 (SEQ ID NO: 3), and Bufo gargarizans Bg-Rsn (SEQ ID NO: 4), more preferably Engystomops pustulosus Ep-Rsn2 (SEQ ID NO: 1) and Leptodactylus vastus Lv-Rsnl (SEQ ID NO: 2). Prefereably the latherins have at least 40%, preferably at least 50%, preferably at least 60%, preferably at least 70%, preferably at least 80%, preferably at least 85%, preferably at least 90%, preferably at least 95%, preferably at least 98% or even 100% amino acid identity to Equus caballus latherin (SEQ ID NO: 10).

## Surfactant System

The surfactant system comprises one or more anionic surfactants and one or more co-surfactants and wherein the weight ratio of said anionic surfactants to said co-surfactants is less than 9:1, more preferably from 5:1 to 1:1, more preferably from 4:1 to 2:1.

Preferably the detergent composition of the invention comprises from 1% to 60%, preferably from 5% to 50%, more preferably from 8% to 40%, by weight of the total composition of a surfactant system.

The surfactant system of the composition of the present invention comprises one or more anionic surfactant. Preferably, the surfactant system for the cleaning composition of the present invention comprises from 1% to 40%, preferably 6% to 35%, more preferably 8% to 30% by weight of the total composition of an anionic surfactant. The anionic surfactant can be any anionic cleaning surfactant, preferably selected from sulfate and/or sulfonate anionic surfactants. HLAS (linear alkylbenzene sulfonate) would be the most preferred sulfonate anionic surfactant. Especially preferred anionic surfactant is selected from the group consisting of alkyl sulfate, alkyl alkoxy sufate and mixtures thereof, and preferably wherein the alkyl alkoxy sulfate is an alkyl ethoxy sulfate. Preferred anionic surfactant is a combination of alkyl sulfates and alkyl ethoxy sulfates with a combined mol average ethoxylation degree of less than 5, preferably less than 3, more preferably less than 2 and more than 0.5 and an average level of branching of from 5% to 40%, more preferably from 10% to 35%, and even more preferably from 20% to 30%.

The average alkoxylation degree is the mol average alkoxylation degree of all the components of the mixture (*i.e.,* mol average alkoxylation degree) of the anionic surfactant. In the mol average alkoxylation degree calculation the weight of sulfate anionic surfactant components not having alkoxylate groups should also be included.

$$\text{Mol average alkoxylation degree} = (x1 * \text{alkoxylation degree of surfactant } 1 + x2 * \text{alkoxylation degree of surfactant } 2 + \ldots) / (x1 + x2 + \ldots)$$

wherein x1, x2, ... are the number of moles of each sulfate anionic surfactant of the mixture and alkoxylation degree is the number of alkoxy groups in each sulfate anionic surfactant.

The average level of branching is the weight average % of branching and it is defined according to the following formula:

$$\text{Weight average of branching (\%)} = [(x1 * \text{wt\% branched alcohol 1 in alcohol } 1 + x2 * \text{wt\% branched alcohol 2 in alcohol } 2 + \ldots) / (x1 + x2 + \ldots)] * 100$$

wherein x1, x2, ... are the weight in grams of each alcohol in the total alcohol mixture of the alcohols which were used as starting material for the anionic surfactant for the composition of the invention. In the weight average branching degree calculation the weight of anionic surfactant components not having branched groups should also be included.

Suitable examples of commercially available sulfates include, those based on Neodol alcohols ex the Shell company, Lial - Isalchem and Safol ex the Sasol company, natural alcohols ex The Procter & Gamble Chemicals company. Suitable sulfonate surfactants for use herein include water-soluble salts of C8-C18 alkyl or hydroxyalkyl sulfonates; C11-C18 alkyl benzene sulfonates (LAS), modified alkylbenzene sulfonate (MLAS); methyl ester sulfonate (MES); and alpha-olefin sulfonate (AOS). Those also include the paraffin sulfonates may be monosulfonates and/or disulfonates, obtained by sulfonating paraffins of 10 to 20 carbon atoms. The sulfonate surfactant also include the alkyl glyceryl sulfonate surfactants.

The surfactant system of the composition of the present invention further comprises one or more co-surfactants, preferably a primary co-surfactant system, wherein the primary co-surfactant system is preferably selected from the group consisting of amphoteric surfactant, zwitterionic surfactant and mixtures thereof. Preferably, the surfactant system for the cleaning composition of the present invention comprises from 0.5% to 15%, preferably from 1% to 12%, more preferably from 2% to 10%, by weight of the total composition of a primary co-surfactant system.

Preferably the primary co-surfactant system is an amphoteric surfactant. Preferably, the primary co-surfactant system is an amine oxide surfactant, and wherein the composition comprises anionic surfactant and amine oxide surfactant in a ratio of less than 9:1, more preferably from 5:1 to 1:1, more preferably from 4:1 to 2:1, preferably from 3:1 to 2.5:1. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or branched alkyl moiety.

Preferably the amine oxide surfactant is a mixture of amine oxides comprising a low-cut amine oxide and a mid-cut amine oxide. The amine oxide of the composition of the invention then comprises:

a) from 10% to 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls or mixtures thereof; and

b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl. In another preferred low-cut amine oxide for use herein R1 and R2 are both methyl. In an especially preferred low-cut amine oxide for use herein R1 and R2 are both methyl and R3 is n-decyl.

Preferably, the amine oxide comprises less than 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Compositions comprising R7R8R9AO tend to be unstable and do not provide very suds mileage.

Preferably the primary co-surfactant system is a zwitterionic surfactant. Suitable exampes of zwitterionic surfactants include betaines, such as alkyl betaines, alkylamidobetaine, amidazoliniumbetaine, sulfobetaine (INCI Sultaines) as well as the Phosphobetaine and preferably meets formula (I):

R1-[CO-X (CH2)n]x-N+(R2)(R3)-(CH2)m-[CH(OH)-CH2]y-Y- (I)

wherein

R1 is a saturated or unsaturated C6-22 alkyl residue, preferably C8-18 alkyl residue, in particular a saturated C10-16 alkyl residue, for example a saturated C12-14 alkyl residue;
X is NH, NR4 with CI-4 Alkyl residue R4, O or S;
n is a number from 1 to 10, preferably 2 to 5, in particular 3;
x is 0 or 1, preferably 1;
R2 and R3 are independently a C1-4 alkyl residue, potentially hydroxy substituted such as a hydroxyethyl, preferably a methyl;
m is a number from 1 to 4, in particular 1, 2 or 3;
y is 0 or 1; and
Y is COO, SO3, OPO(OR5)O or P(O)(OR5)O, whereby R5 is a hydrogen atom H or a C1-4 alkyl residue.

Preferred betaines are the alkyl betaines of the formula (Ia), the alkyl amido propyl betaine of the formula (Ib), the Sulfo betaines of the formula (Ic), and the Amido sulfobetaine of the formula (Id);

R1-N+(CH3)2-CH2COO- (Ia)

R1-CO-NH(CH2)3-N+(CH3)2-CH2COO- (Ib)

R1-N+(CH3)2-CH2CH(OH)CH2SO3- (Ic)

R1-CO-NH-(CH2)3-N+(CH3)2-CH2CH(OH)CH2SO3- (Id)

in which R1 has the same meaning as in formula I. Particularly preferred betaines are the Carbobetaine [wherein Y-=COO-], in particular the Carbobetaine of the formula (Ia) and (Ib), more preferred are the Alkylamidobetaine of the formula (Ib). A preferred betaine is, for example, Cocoamidopropylbetaine.

Preferably the surfactant system of the composition of the present invention further comprises from 0.1% to 10% by weight of the total composition of a secondary co-surfactant system preferably comprising a non-ionic surfactant. Suitable non-ionic surfactants include the condensation products of aliphatic alcohols with from 1 to 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from 10 to 18 carbon atoms, preferably from 10 to 15 carbon atoms with from 2 to 18 moles, preferably 2 to 15, more preferably 5-12 of ethylene oxide per mole of alcohol. Highly preferred non-ionic surfactants are the condensation products of guerbet alcohols with from 2 to 18 moles, preferably 2 to 15, more preferably 5-12 of ethylene oxide per mole of alcohol. Preferably, the non-ionic surfactants are an alkyl ethoxylated surfactants, preferably comprising from 9 to 15 carbon atoms in its alkyl chain and from 5 to 12 units of ethylene oxide per mole of alcohol. Other suitable non-ionic surfactants for use herein include fatty alcohol polyglycol ethers, alkylpolyglucosides and fatty acid glucamides, preferably alkylpolyglucosides. Preferably the alkyl polyglucoside surfactant is a C8-C16 alkyl polyglucoside surfactant, preferably a C8-C14 alkyl polyglucoside surfactant, preferably with an average degree of polymerization of between 0.1 and 3, more preferably between 0.5 and 2.5, even more preferably between 1 and 2. Most preferably the alkyl polyglucoside surfactant has an average alkyl carbon chain length between 10 and 16, preferably between 10 and 14, most preferably between 12 and 14, with an average degree of polymerization of between 0.5 and 2.5 preferably between 1 and 2, most preferably between 1.2 and 1.6. C8-C16 alkyl polyglucosides are commercially available from several suppliers (e.g., Simusol® surfactants from Seppic Corporation; and Glucopon® 600 CSUP, Glucopon® 650 EC, Glucopon® 600 CSUP/MB, and Glucopon® 650 EC/MB, from BASF Corporation). Preferably, the composition comprises the anionic surfactant and the non-ionic surfactant in a ratio of from 2:1 to 50:1, preferably 2:1 to 10:1.

## Enzymes

Preferred compositions of the invention comprise one or more enzymes selected from the group consisting of amylases, lipases, proteases, cellulases, lipoxygenases, diol synthases, and mixtures thereof. Each additional enzyme is typically present in an amount from 0.0001 wt% to 1 wt% (based on active protein) more preferably from 0.0005 wt% to 0.5 wt%, most preferably 0.005 wt% to 0.1 wt%, by weight of the cleaning composition.

## Enzyme Stabilizer

Preferably the composition of the invention comprises an enzyme stabilizer. Suitable enzyme stabilizers may be selected from the group consisting of (a) univalent, bivalent and/or trivalent cations preferably selected from the group of inorganic or organic salts of alkaline earth metals, alkali metals, aluminum, iron, copper and zinc, preferably alkali metals and alkaline earth metals, preferably alkali metal and alkaline earth metal salts with halides, sulfates, sulfites, carbonates, hydrogencarbonates, nitrates, nitrites, phosphates, formates, acetates, propionates, citrates, maleates, tartrates, succinates, oxalates, lactates, and mixtures thereof. Preferably the salt is selected from the group consisting of sodium chloride, calcium chloride, potassium chloride, sodium sulfate, potassium sulfate, sodium acetate, potassium acetate, sodium formate, potassium formate, calcium lactate, calcium nitrate and mixtures thereof. Most preferred are salts selected from the group consisting of calcium chloride, potassium chloride, potassium sulfate, sodium acetate, potassium acetate, sodium formate, potassium formate, calcium lactate, calcium nitrate, and mixtures thereof, and in particular potassium salts selected from the group of potassium chloride, potassium sulfate, potassium acetate, potassium formate, potassium propionate, potassium lactate and mixtures thereof. Most preferred are potassium acetate and potassium chloride. Preferred calcium salts are calcium formate, calcium lactate and calcium nitrate including calcium nitrate tetrahydrate. Calcium and sodium formate salts may be preferred. These cations are present at at least 0.01 wt%, preferably at least 0.03 wt%, more preferably at least 0.05 wt%, most preferably at least 0.25 wt% up to about 2 wt% or even up to 1 wt% by weight of the total composition. These salts are formulated from 0.1 to 5 wt%, preferably from 0.2 to 4 wt%, more preferably from 0.3 to 3 wt%, most preferably from 0.5 to 2 wt% relative to the total weight of the composition. Further enzyme stabilizers can be selected from the group (b) carbohydrates selected from the group

consisting of oligosaccharides, polysaccharides and mixtures thereof, such as a monosaccharide glycerate as described in WO201219844; (c) mass efficient reversible protease inhibitors selected from the group consisting of phenyl boronic acid and derivatives thereof, preferably 4-formyl phenylboronic acid; (d) alcohols such as 1,2-propane diol, propylene glycol; (e) peptide aldehyde stabilizers such as tripeptide aldehydes such as Cbz-Gly-Ala-Tyr-H, or disubstituted alaninamide; (f) carboxylic acids such as phenyl alkyl dicarboxylic acid as described in WO2012/19849 or multiply substituted benzyl carboxylic acid comprising a carboxyl group on at least two carbon atoms of the benzyl radical such as described in WO2012/19848, phthaloyl glutamine acid, phthaloyl asparagine acid, aminophthalic acid and/or an oligoamino-biphenyl-oligocarboxylic acid; and; (g) mixtures thereof.

Salt

The composition of the present invention may optionally comprise from 0.01% to 3%, preferably from 0.05% to 2%, more preferably from 0.2% to 1.5%, or most preferably 0.5% to 1%, by weight of the total composition of a salt, preferably a monovalent, divalent inorganic salt or a mixture thereof, preferably sodium chloride. Most preferably the composition alternatively or further comprises a multivalent metal cation in the amount of from 0.01 wt% to 3 wt%, preferably from 0.05% to 2%, more preferably from 0.2% to 1.5%, or most preferably 0.5% to 1% by weight of said composition, preferably said multivalent metal cation is magnesium, aluminium, copper, calcium or iron, more preferably magnesium, most preferably said multivalent salt is magnesium chloride. Without wishing to be bound by theory, it is believed that use of a multivalent cation helps with the formation of protein/ protein, surfactant/ surfactant or hybrid protein/ surfactant network at the oil water and air water interface that is strengthening the suds.

Carbohydrates

Preferably the composition of the present invention comprises one or more carbohydrates selected from the group comprising O-glycan, N-glycan, and mixtures thereof. Preferably the cleaning composition further comprises one or more carbohydrates selected from the group comprising derivatives of glucose, mannose, lactose, galactose, allose, altrose, gulose, idose, talose, fucose, fructose, sorbose, tagatose, psicose, arabinose, ribose, xylose, lyxose, ribulose, and xylulose. More preferably the cleaning composition comprises one or more carbohydrates selected from the group of $\alpha$-glucans and $\beta$-glucans. Glucans are polysaccharides of D-glucose monomers, linked by glycosidic bonds. Non-limiting examples of $\alpha$-glucans are dextran, starch, floridean starch, glycogen, pullulan, and their derivatives. Non-limiting examples of $\beta$-glucans are cellulose, chrysolaminarin, curdlan, laminarin, lentinan, lichenin, oat beta-glucan, pleuran, zymosan, and their derivatives.

Hydrotrope

The composition of the present invention may optionally comprise from 1% to 10%, or preferably from 0.5% to 10%, more preferably from 1% to 6%, or most preferably from 0.1% to 3%, or combinations thereof, by weight of the total composition of a hydrotrope, preferably sodium cumene sulfonate. Other suitable hydrotropes for use herein include anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in U.S. Patent 3,915,903. Preferably the composition of the present invention is isotropic. An isotropic composition is distinguished from oil-in-water emulsions and lamellar phase compositions. Polarized light microscopy can assess whether the composition is isotropic. See e.g., The Aqueous Phase Behaviour of Surfactants, Robert Laughlin, Academic Press, 1994, pp. 538-542. Preferably an isotropic composition is provided. Preferably the composition comprises 0.1% to 3% by weight of the total composition of a hydrotrope, preferably wherein the hydrotrope is selected from sodium, potassium, and ammonium xylene sulfonate, sodium, potassium and ammonium toluene sulfonate, sodium potassium and ammonium cumene sulfonate, and mixtures thereof.

Organic solvent

The composition of the present invention may optionally comprise an organic solvent. Suitable organic solvents include C4-14 ethers and diethers, polyols, glycols, alkoxylated glycols, C6-C16 glycol ethers, alkoxylated aromatic alcohols, aromatic alcohols, aliphatic linear or branched alcohols, alkoxylated aliphatic linear or branched alcohols, alkoxylated C1-C5 alcohols, C8-C14 alkyl and cycloalkyl hydrocarbons and halohydrocarbons, and mixtures thereof. Preferably the organic solvents include alcohols, glycols, and glycol ethers, alternatively alcohols and glycols. The composition comprises from 0% to less than 50%, preferably from 0.01% to 25%, more preferably from 0.1% to 10%, or most preferably from 0.5% to 5%, by weight of the total composition of an organic solvent, preferably an alcohol, more preferably an ethanol, a polyalkyleneglycol, more preferably polypropyleneglycol, and mixtures thereof.

Amphiphilic Polymer

The composition of the present invention may further comprise from 0.01% to 5%, preferably from 0.05% to 2%, more preferably from 0.07% to 1% by weight of the total composition of an amphiphilic polymer selected from the groups consisting of amphiphilic alkoxylated polyalkyleneimine and mixtures thereof, preferably an amphiphilic alkoxylated polyalkyleneimine.

Preferably, the amphiphilic alkoxylated polyalkyleneimine is an alkoxylated polyethyleneimine polymer comprising a polyethyleneimine backbone having average molecular weight range from 100 to 5,000, preferably from 400 to 2,000, more preferably from 400 to 1,000 Daltons and the alkoxylated polyethyleneimine polymer further comprising:

(i) one or two alkoxylation modifications per nitrogen atom by a polyalkoxylene chain having an average of 1 to 50 alkoxy moieties per modification, wherein the terminal alkoxy moiety of the alkoxylation modification is capped with hydrogen, a C1-C4 alkyl or mixtures thereof;
(ii) an addition of one C1-C4 alkyl moiety and one or two alkoxylation modifications per nitrogen atom by a polyalkoxylene chain having an average of 1 to 50 alkoxy moieties per modification wherein the terminal alkoxy moiety is capped with hydrogen, a C1-C4 alkyl or mixtures thereof; or
(iii)a combination thereof; and

wherein the alkoxy moieties comprises ethoxy (EO) and/or propxy (PO) and/or butoxy (BO) and wherein when the alkoxylation modification comprises EO it also comprises PO or BO.

Preferred amphiphilic alkoxylated polyethyleneimine polymers comprise EO and PO groups within their alkoxylation chains, the PO groups preferably being in terminal position of the alkoxy chains, and the alkoxylation chains preferably being hydrogen capped. Hydrophilic alkoxylated polyethyleneimine polymers solely comprising ethoxy (EO) units within the alkoxylation chain could also optionally be formulated within the scope of this invention.

For example, but not limited to, below is shown possible modifications to terminal nitrogen atoms in the polyethyleneimine backbone where R represents an ethylene spacer and E represents a C1-C4 alkyl moiety and X- represents a suitable water soluble counterion.

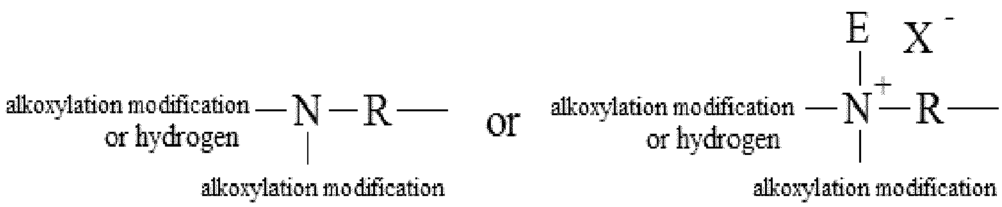

Also, for example, but not limited to, below is shown possible modifications to internal nitrogenatoms in the polyethyleneimine backbone where R represents an ethylene spacer and E represents a $C_1$-$C_4$ alkyl moiety and X- represents a suitable water soluble counterion.

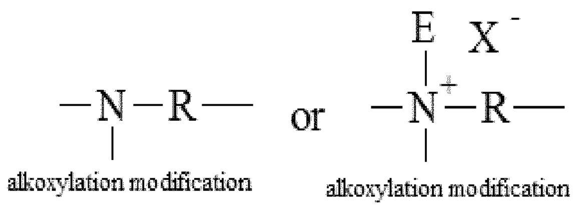

The alkoxylation modification of the polyethyleneimine backbone consists of the replacement of a hydrogen atom by a polyalkoxylene chain having an average of 1 to 50 alkoxy moieties, preferably from 20 to 45 alkoxy moieties, most preferably from 30 to 45 alkoxy moieties. The alkoxy moieties are selected from ethoxy (EO), propoxy (PO),butoxy (BO), and mixtures thereof. Alkoxy moieties solely comprising ethoxy units are outside the scope of the invention though. Preferably, the polyalkoxylene chain is selected from ethoxy/propoxy block moieties. More preferably, the polyalkoxylene chain is ethoxy/propoxy block moieties having an average degree of ethoxylation from 3 to 30 and an average degree of propoxylation from 1 to 20, more preferably ethoxy/propoxy block moieties having an average degree of ethoxylation from 20 to 30 and an average degree of propoxylation from 10 to 20.

More preferably the ethoxy/propoxy block moieties have a relative ethoxy to propoxy unit ratio between 3 to 1 and 1 to 1, preferably between 2 to 1 and 1 to 1. Most preferably the polyalkoxylene chain is the ethoxy/propoxy block

moieties wherein the propoxy moiety block is the terminal alkoxy moiety block.

The modification may result in permanent quaternization of the polyethyleneimine backbone nitrogen atoms. The degree of permanent quaternization may be from 0% to 30% of the polyethyleneimine backbone nitrogen atoms. It is preferred to have less than 30% of the polyethyleneimine backbone nitrogen atoms permanently quaternized. Most preferably the degree of quaternization is 0%.

A preferred polyethyleneimine has the general structure of Formula (II):

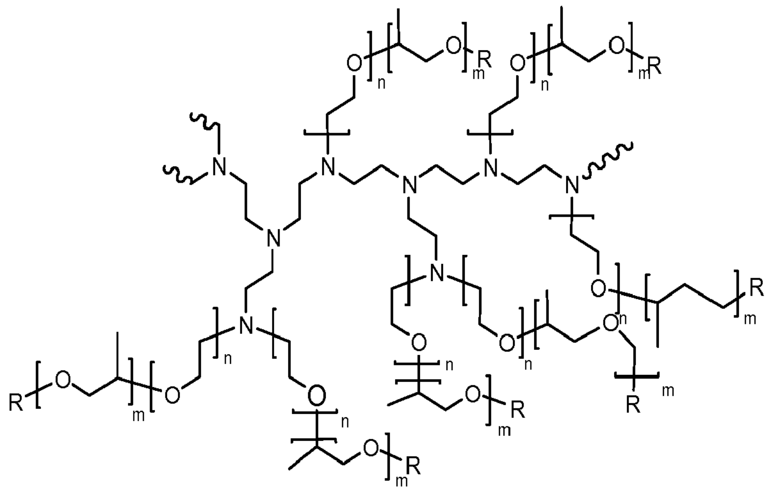

(II)

wherein the polyethyleneimine backbone has a weight average molecular weight of 600, n of formula (II) has an average of 10, m of formula (II) has an average of 7 and R of formula (II) is selected from hydrogen, a $C_1$-$C_4$ alkyl and mixtures thereof, preferably hydrogen. The degree of permanent quaternization of formula (II) may be from 0% to 22% of the polyethyleneimine backbone nitrogen atoms. The molecular weight of this polyethyleneimine preferably is between 10,000 and 15,000.

An alternative polyethyleneimine has the general structure of Formula (II) but wherein the polyethyleneimine backbone has a weight average molecular weight of 600, n of Formula (II) has an average of 24, m of Formula (II) has an average of 16 and R of Formula (II) is selected from hydrogen, a $C_1$-$C_4$ alkyl and mixtures thereof, preferably hydrogen. The degree of permanent quaternization of Formula (II) may be from 0% to 22% of the polyethyleneimine backbone nitrogen atoms. The molecular weight of this polyethyleneimine preferably is between 25,000 and 30,000.

Most preferred polyethyleneimine has the general structure of Formula (II) wherein the polyethyleneimine backbone has a weight average molecular weight of 600, n of Formula (II) has an average of 24, m of Formula (II) has an average of 16 and R of Formula (II) is hydrogen. The degree of permanent quaternization of Formula (II) is 0% of the polyethyleneimine backbone nitrogen atoms. The molecular weight of this polyethyleneimine preferably is from 25,000 to 30,000, most preferably 28,000.

These polyethyleneimines can be prepared, for example, by polymerizing ethyleneimine in the presence of a catalyst such as carbon dioxide, sodium bisulfite, sulfuric acid, hydrogen peroxide, hydrochloric acid, acetic acid, and the like, as described in more detail in PCT Publication No. WO 2007/135645.

Chelant

The detergent composition herein can comprise a chelant at a level of from 0.1% to 20%, preferably from 0.2% to 5%, more preferably from 0.2% to 3% by weight of total composition.

As commonly understood in the detergent field, chelation herein means the binding or complexation of a bi- or multidentate ligand. These ligands, which are often organic compounds, are called chelants, chelators, chelating agents, and/or sequestering agent. Chelating agents form multiple bonds with a single metal ion. Chelants, are chemicals that form soluble, complex molecules with certain metal ions, inactivating the ions so that they cannot normally react with other elements or ions to produce precipitates or scale, or forming encrustations on soils turning them harder to be removed. The ligand forms a chelate complex with the substrate. The term is reserved for complexes in which the metal ion is bound to two or more atoms of the chelant.

Preferably, the composition of the present invention comprises one or more chelant, preferably selected from the group comprising carboxylate chelants, amino carboxylate chelants, amino phosphonate chelants such as MGDA (methylglycine-N,N-diacetic acid), GLDA (glutamic-N,N- diacetic acid), and mixtures thereof.

Suitable chelating agents can be selected from the group consisting of amino carboxylates, amino phosphonates, polycarboxylate chelating agents and mixtures thereof.

Other chelants include homopolymers and copolymers of polycarboxylic acids and their partially or completely neutralized salts, monomeric polycarboxylic acids and hydroxycarboxylic acids and their salts. Suitable polycarboxylic acids are acyclic, alicyclic, heterocyclic and aromatic carboxylic acids, in which case they contain at least two carboxyl groups which are in each case separated from one another by, preferably, no more than two carbon atoms. A suitable hydroxycarboxylic acid is, for example, citric acid. Another suitable polycarboxylic acid is the homopolymer of acrylic acid. Preferred are the polycarboxylates end capped with sulfonates.

Adjunct Ingredients

The cleaning composition herein may optionally comprise a number of other adjunct ingredients such as builders (*e.g.*, preferably citrate), cleaning solvents, cleaning amines, conditioning polymers, cleaning polymers, surface modifying polymers, soil flocculating polymers, structurants, emollients, humectants, skin rejuvenating actives, enzymes, carboxylic acids, scrubbing particles, bleach and bleach activators, perfumes, malodor control agents, pigments, dyes, opacifiers, beads, pearlescent particles, microcapsules, inorganic cations such as alkaline earth metals such as Ca/Mg-ions, antibacterial agents, preservatives, viscosity adjusters (*e.g.,* salt such as NaCl, and other mono-, di- and trivalent salts) and pH adjusters and buffering means (*e.g.,* carboxylic acids such as citric acid, HCl, NaOH, KOH, alkanolamines, phosphoric and sulfonic acids, carbonates such as sodium carbonates, bicarbonates, sesquicarbonates, borates, silicates, phosphates, imidazole and alike).

Method of Washing

The present invention is directed to a method comprising contacting a cleaning composition with a surface, wherein the composition comprises one or more surface active proteins selected from the group consisting of ranaspumins, latherins, and mixtures thereof, preferably ranaspumins, and a specific surfactant system. As such, the composition herein will be applied in its diluted form to the dishware. Soiled surfaces *e.g.* dishes are contacted with an effective amount, typically from 0.5 mL to 20 mL (per 25 dishes being treated), preferably from 3mL to 10 mL, of the detergent composition of the present invention, preferably in liquid form, diluted in water. The actual amount of detergent composition used will be based on the judgment of user, and will typically depend upon factors such as the particular product formulation of the composition, including the concentration of active ingredients in the composition, the number of soiled dishes to be cleaned, the degree of soiling on the dishes, and the like. Generally, from 0.01 mL to 150 mL, preferably from 3 mL to 40 mL of a liquid detergent composition of the invention is combined with from 2,000 mL to 20,000 mL, more typically from 5,000 mL to 15,000 mL of water in a sink having a volumetric capacity in the range of from 1,000 mL to 20,000 mL, more typically from 5,000 mL to 15,000 mL. The soiled dishes are immersed in the sink containing the diluted compositions then obtained, where contacting the soiled surface of the dish with a cloth, sponge, or similar article cleans them. The cloth, sponge, or similar article may be immersed in the detergent composition and water mixture prior to being contacted with the dish surface, and is typically contacted with the dish surface for a period of time ranged from 1 to 10 seconds, although the actual time will vary with each application and user. The contacting of cloth, sponge, or similar article to the surface is preferably accompanied by a concurrent scrubbing of the surface.

In another aspect, the invention is directed to a method of manually washing soiled articles preferably dishware comprising contacting a cleaning composition with a surface preferably dishware, wherein the composition comprises one or more surface active proteins selected from the group consisting of ranaspumins, latherins, and mixtures thereof, preferably ranaspumins, and a specific surfactant system, wherein said composition modifies the hydrophobicity of said surface preferably dishware as a result of said contacting step.

Another aspect of the present invention is directed to a method of promoting suds longevity or grease emulsification in a washing process for washing soiled articles, preferably dishware. The method comprises the steps of: a) delivering a cleaning composition comprising one or more surface active proteins selected from the group consisting of ranaspumins, latherins, and mixtures thereof, preferably ranaspumins, and a specific surfactant system to a volume of water to form a wash liquor; and b) immersing the soiled articles into said wash liquor. Preferably, the surface active proteins are present at a concentration of 0.005 ppm to 60 ppm, preferably at a concentration of 0.02 ppm to 12 ppm, based on active protein, in an aqueous wash liquor during the washing process.

Another aspect of the present invention is directed to use of surface active proteins to provide increased suds longevity or increased grease emulsification in an aqueous wash liquor during a washing process, wherein said surface active proteins are selected from the group consisting of ranaspumins, latherins, and mixtures thereof, preferably ranaspumins.

Preferably the ranaspumins have at least 40%, preferably at least 50%, preferably at least 60%, preferably at least 70%, preferably at least 80%, preferably at least 85%, preferably at least 90%, preferably at least 95%, preferably

at least 98% or even 100% amino acid identity to at least one wild-type protein selected from the group consisting of: *Engystomops pustulosus* Ep-Rsn2 (SEQ ID NO: 1), *Leptodactylus vastus* Lv-Rsnl (SEQ ID NO: 2), *Leptodactylus fuscus* Lf-Rsn1 (SEQ ID NO: 3), and *Bufo gargarizans* Bg-Rsn (SEQ ID NO: 4), and the latherins have at least 40%, preferably at least 50%, preferably at least 60%, preferably at least 70%, preferably at least 80%, preferably at least 85%, preferably at least 90%, preferably at least 95%, preferably at least 98% or even 100% amino acid identity to *Equus caballus* latherin (SEQ ID NO: 10).

Preferably the aqueous wash liquor comprises a surfactant system, wherein the surfactant system comprises one or more anionic surfactants and one or more co-surfactants and wherein the weight ratio of the anionic surfactants to the co-surfactants is less than 9:1, more preferably from 5:1 to 1:1, more preferably from 4:1 to 2:1.

TEST METHODS

The following assays set forth must be used in order that the invention described and claimed herein may be more fully understood.

Test Method 1 - Glass Vial Suds Mileage Method

The objective of the glass vial suds mileage test method is to measure the evolution of suds volume over time generated by a certain solution of detergent composition in the presence of a greasy soil, *e.g.,* olive oil. The steps of the method are as follows:

1. Test solutions are prepared by subsequently adding aliquots at room temperature of: a) 10 g of an aqueous detergent solution at specified detergent concentration and water hardness, b) 1.0 g of an aqueous protein solution at specified concentration and water hardness, and c) 0.11 g of olive oil (Bertolli®, Extra Virgin Olive Oil), into a 40 mL glass vial (dimensions: 95 mm H x 27.5 mm D). For the reference samples, the protein solutions are substituted with 1.0 mL of demineralized water. For the nil detergent samples, the 10g of aqueous detergent solution is replaced by 10 g of water at specified water hardness.
2. The test solutions are mixed in the closed test vials by stirring at room temperature for 2 minutes on a magnetic stirring plate (IKA, model # RTC B S001; VWR magnetic stirrer, catalog # 58949-012; 500 RPM), followed by manually shaking for 20 seconds with an upwards downwards movement (about 2 up and down cycles per second, +/- 30 cm up and 30 cm down).
3. Following the shaking, the test solutions in the closed vials are further stirred on a magnetic stirring plate (IKA, model # RTC B S001; VWR magnetic stirrer, catalog # 58949-012; 500 RPM) for 60 minutes inside a water bath at 46 °C to maintain a constant temperature. The samples are then shaken manually for another 20 seconds as described above and the initial suds heights (H1) are recorded with a ruler.
4. The samples are incubated for an additional 30 minutes inside the water bath at 46 °C while stirring (IKA, model # RTC B S001; VWR magnetic stirrer, catalog # 58949-012; 500 RPM), followed by manual shaking for another 20 seconds as described above. The final suds heights (H2) are recorded.
5. Protein solutions that produce larger suds heights (HI and H2), preferably combined with lower drops in suds height between H1 and H2, are more desirable.

Test Method 2 - Sink Suds Mileage Method

The evolution of the suds volume generated by a solution of a detergent composition can be determined while adding soil loads periodically as follows. A stream of hard water (15 dH) fills a sink (cylinder dimensions: 300 mm D x 288 mm H) to 4 L with a constant pressure of 4 bar. Simultaneously, an aliquot of the detergent composition (final concentration 0.12 wt%) is dispensed through a pipette with a flow rate of 0.67 mL/ sec at a height of 37 cm above the bottom of the sink surface. An initial suds volume is generated in the sink due to the pressure of the water. The temperature of the solution is maintained at 46 °C during the test.

After recording the initial suds volume (average suds height x sink surface area), a fixed amount of greasy soil (Composition : see Table 1, 6 mL) is injected in the middle of the sink, while a paddle (dimensions: 10 cm x 5 cm, positioned in the middle of the sink at the air liquid interface at an angle of 45 degrees) rotates 20 times into the solution at 85 RPM. This step is followed immediately by another measurement of the total suds volume. The soil injecting, paddling, and measuring steps are repeated until the measured suds volume reaches a minimum level, which is set at 400 $cm^3$. The amount of soil additions needed to get to that level is recorded. The complete process is repeated a number of times and the average of the number of additions for all the replicates is calculated for each detergent composition

Finally, the suds mileage index is then calculated as: (average number of soil additions for test detergent

composition) / (average number of soil additions for reference detergent composition) x 100.

Pending on the test purpose the skilled person could choose to select an alternative water hardness, solution temperature, product concentration or soil type.

Table 1: Greasy Soil Composition

| Ingredient | Weight % |
|---|---|
| Crisco oil | 12.730 |
| Crisco shortening | 27.752 |
| Lard | 7.638 |
| Refined Rendered Edible Beef Tallow | 51.684 |
| Oleic Acid, 90% (Techn) | 0.139 |
| Palmitic Acid, 99+% | 0.036 |
| Stearic Acid, 99+% | 0.021 |

EXAMPLES

The following examples are provided to further illustrate the present invention and are not to be construed as limitations of the present invention, as many variations of the present invention are possible without departing from its spirit or scope.

Example 1a - Production of *Engystomops pustulosus* Ep-Rsn2

A codon optimized gene (SEQ ID NO: 11) encoding for an *Engystomops pustulosus* Ep-Rsn2 variant, including an N-terminal His-tag and a TEV protease cleavage site (SEQ ID NO: 12), is designed and synthesized and the protein is expressed and purified by Genscript (Piscataway, NJ). In brief, the complete synthetic gene sequence is subcloned into a pET30a vector for heterologous expression. *Escherichia coli* BL21 (DE3) cells are transformed with the recombinant plasmid and a single colony was inoculated into TB medium containing the proper kanamycin. Isopropyl β-D-1-thiogalactopyranoside (IPTG) is added (final concentration 0.1 mM) to induce protein expression and the culture is incubated at 15 °C and 200 rpm for 16 hrs. Cells are harvested by centrifugation and the pellet is lysed by sonication. After centrifugation, the supernatant is collected and the protein is purified by one-step purification using a nickel affinity column and standard protocols known in the art. The protein is stored in a buffer containing 50 mM Tris-HCl, 150 mM NaCl, and 10% Glycerol at pH 8.0. The final protein concentration is 1.25 mg/ mL as determined by Bradford protein assay with BSA as a standard (ThermoFisher, catalog # 23236).

Example 1b - Production of *Leptodactylus vastus* Lv-Rsnl

A codon optimized gene (SEQ ID NO: 13) encoding for a *Leptodactylus vastus* Lv-Rsnl variant, including an N-terminal His-tag, and a TEV protease cleavage site (SEQ ID NO: 14), is designed and synthesized and the protein is expressed and purified by Genscript (Piscataway, NJ). In brief, the complete synthetic gene sequence is subcloned into a pPICZalpha-A vector for heterologous expression. The linearized construct is then transformed into *Pichia pastoris* X-33 and the insert of the target gene is confirmed by PCR analysis. Four colonies are inoculated in BMGY for protein expression. When $OD_{600}$ reached 3, the cells are harvested and re-suspended in BMMY media. Methanol is added to a final concentration of 1% every 24 hours for 4 days. After centrifugation, the supernatants are collected and analyzed by SDS-PAGE. The protein is purfied by two-step purification using Ni column and SP Sepharose column and standard protocols known in the art. The protein is stored in a buffer containing 50 mM Tris-HCl, 150 mM NaCl, and 10% Glycerol at pH 8.0. The final protein concentration is 50 μg/ mL as determined by Micro-Bradford protein assay with BSA as a standard (ThermoFisher, catalog # 23236).

Example 1c - Detergent Compositions

The evolution of suds volume generated by a certain solution of detergent composition in presence of a soil, *i.e.,* olive oil or greasy soil, is followed over time under specific conditions (e.g., water hardness, solution temperature, detergent concentrations, etc.). The following solutions are prepared:

A. Hard water (15 dH): 0.75 g $MgCl_2.6H_2O$ (Sigma-Aldrich, catalog # M9272), 2.10 g $CaCl_2.6H_2O$ (Sigma-Aldrich, catalog # 21108), and 0.689 g $NaHCO_3$ (Sigma-Aldrich, catalog # 31437) are dissolved in 5 L of demineralized water.
B. Detergent solution of a high surfactant content detergent composition ("solution DG-HS") is prepared using Fairy Dark Green, as commercially available in the UK in Feb 2017, diluted in hard water (15 dH) prepared as above, at targeted detergent concentration of 0.12%.
C. Detergent solution of a low surfactant content detergent composition ("solution DG-LS") is prepared using Fairy Dark Green, as commercially available in the UK in Feb 2017, diluted in hard water (15 dH) prepared as above, at targeted detergent concentration of 0.06%.
D. Protein solutions: Proteins are diluted in demineralized water to the required concentration before proceeding with the suds mileage method.
E. Greasy soil: A grease soil is prepared according to the composition described in Table 1.

Example 2 - Glass Vial Suds Mileage of *Engystomops pustulosus* Ep-Rsn2 with Olive Oil

Inventive Compositions A, B and C are examples of cleaning compositions according to the present invention, made with: a) detergent solution DG-LS (prepared as described in Example 1c) comprising a surfactant system according to the invention, and b) diluted samples of purified *Engystomops pustulosus* Ep-Rsn2 (SEQ ID NO: 12) (prepared as described in Example 1a), a protein according to the invention. Comparative Composition D contains the same detergent solution DG-LS comprising a surfactant system according to the invention but in the absence of the surface active protein. The glass vial suds mileage test was performed on the compositions using olive oil as described in the test methods section (Test Method 1). The initial (H1) and final (H2) measurements are recorded in Table 2. The % suds height drop represents the drop in suds height as measured between the initial and final time point and is calculated by the following equation:

$$\% \text{ suds height drop} = \{(H1 - H2)/H1\}*100.$$

The % suds height drops are calculated for the compositions and shown in Table 2.

Table 2: Suds Mileage

| Composition | Ep-Rsn2 Concentration in Composition [ppm] | H1 [mm] | H2 [mm] | % suds height drop H2 vs H1 |
|---|---|---|---|---|
| Inventive Composition A | 12 | 5.5 | 5 | 9% |
| Inventive Composition B | 6 | 5.5 | 4.5 | 18% |
| Inventive Composition C | 1.2 | 5.5 | 3 | 45% |
| Comparative Composition D | 0 | 4 | 2 | 50% |

The results confirm that Inventive Compositions A-C detergent solutions comprising *Engystomops pustulosus* Ep-Rsn2 (SEQ ID NO: 12) protein according to the invention and a specific surfactant system according to the invention have a superior suds profile compared to Comparative Composition D solution comprising a surfactant system according to the invention but without the surface active protein, both in view of absolute suds height build-up as in view of sustaining the suds height in presence of greasy soil.

A further Comparative Composition H is an example of a cleaning composition outside the scope of the present invention, made with a diluted sample (12 ppm in composition) of *Engystomops pustulosus* Ep-Rsn2 (SEQ ID NO: 12) protein according to the invention in the absence of the detergent solution DG-LS (replaced with hard water - 15 dH). The glass vial suds mileage test was performed on Comparative Composition H using olive oil as described in the test methods section (Test Method 1), but did not show any sudsing (HI and H2 equal zero) (data not shown), illustrating a synergistic suds build up and maintenance boost when combining the specific protein with the specific surfactant system according to the invention.

Example 3 - Glass Vial Suds Mileage of *Leptodactylus vastus* Lv-Rsnl with Olive Oil

Inventive Compositions E and F are examples of cleaning compositions according to the present invention, made with: a) detergent solution DG-LS (prepared as described in Example 1c) comprising a surfactant system according to the invention, and b) diluted samples of purified *Leptodactylus vastus* Lv-Rsnl (SEQ ID NO: 14) (prepared as described in Example 1b) protein according to the invention. Comparative Composition G contains the same detergent solution DG-LS comprising the surfactant system according to the invention but in the absence of the surface active protein. The glass vial suds mileage test was performed on these compositions using olive oil as described in the test methods section (Test Method 1). The initial (H1) and final (H2) measurements are recorded in Table 3. The % suds height drop are calculated for the compositions and are shown in Table 3.

Table 3: Suds Mileage

| Composition | Lv-Rsn1 Concentration in Composition [ppm] | H1 [mm] | H2 [mm] | % suds height drop H2 vs H1 |
|---|---|---|---|---|
| Inventive Composition E | 3.6 | 8 | 7 | 12% |
| Inventive Composition F | 1.2 | 7.5 | 6 | 20% |
| Comparative Composition G | 0 | 5 | 2.5 | 50% |

The results confirm that Inventive Compositions E and F detergent solutions comprising *Leptodactylus vastus* Lv-Rsnl (SEQ ID NO: 14) protein according to the invention and a surfactant system according to the invention have a superior suds profile compared to Comparative Composition G solution comprising a surfactant system according to the invention but without the surface active protein, both in view of absolute suds height build-up as in view of sustaining the suds height in presence of greasy soil.

A further Comparative Composition I is an example of a cleaning composition outside the scope of the present invention, made with a diluted sample (3.6 ppm in composition) of *Leptodactylus vastus* Lv-Rsnl (SEQ ID NO: 14) protein according to the invention in the absence of the detergent solution DG-LS (replaced with hard water - 15 dH). The glass vial suds mileage test is performed on Comparative Composition H using olive oil as described in the test methods section (Test Method 1), but did not show any sudsing (HI and H2 equal zero), illustrating a synergistic suds build up and maintenance boost when combining the specific protein with the specific surfactant system according to the invention.

Example 4 - Exemplary Manual Dish-Washing Detergent Composition

Table 4 exemplifies a manual dish-washing detergent composition comprising *Engystomops pustulosus* Ep-Rsn2 (SEQ ID NO: 1) or *Leptodactylus vastus* Lv-Rsnl (SEQ ID NO: 2) proteins according to the invention.

Table 4: Detergent Composition

| Ingredient | Wt% |
|---|---|
| Sodium alkyl ethoxy sulfate (C1213E00.6S) | 22.91% |
| n-C12-14 Di Methyl Amine Oxide | 7.64% |
| Lutensol® XP80 (non-ionic surfactant supplied by BASF) | 0.45% |
| Sodium Chloride | 1.2% |
| Poly Propylene Glycol (MW 2000) | 1% |
| Ethanol | 2% |
| Sodium Hydroxide | 0.24% |
| *Engystomops pustulosus* Ep-Rsn2 (SEQ ID NO: 1) or *Leptodactylus vastus* Lv-Rsnl (SEQ ID NO: 2) | 0.5% |
| Minors (perfume, preservative, dye) + water | To 100 % |
| pH (@ 10% solution) | 9 |

All percentages and ratios given for proteins are based on active protein. All percentages and ratios herein are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A cleaning composition comprising:

(a) one or more surface active proteins selected from the group consisting of ranaspumins, latherins, and mixtures thereof, preferably ranaspumins;

(i) wherein the ranaspumins have at least 80%, preferably at least 85%, preferably at least 90%, preferably at least 95%, preferably at least 98%, or even 100% amino acid identity as calculated over the entire length of the sequence aligned against the entire length of at least one reference sequence selected from the group consisting of: *Engystomops pustulosus* Ep-Rsn2 (SEQ ID NO: 1), *Leptodactylus vastus* Lv-Rsn1 (SEQ ID NO: 2), *Leptodactylus fuscus* Lf-Rsn1 (SEQ ID NO: 3), and *Bufo gargarizans* Bg-Rsn (SEQ ID NO: 4), preferably *Engystomops pustulosus* Ep-Rsn2 (SEQ ID NO: 1) and *Leptodactylus vastus* Lv-Rsn1 (SEQ ID NO: 2); and
(ii) wherein the latherins have at least 80%, preferably at least 85%, preferably at least 90%, preferably at least 95%, preferably at least 98%, or even 100% amino acid identity as calculated over the entire length of the sequence aligned against the entire length of *Equus caballus* latherin (SEQ ID NO: 10); and

(b) a surfactant system, wherein the surfactant system comprises one or more anionic surfactants and one or more co-surfactants and wherein the weight ratio of the anionic surfactants to the co-surfactants is less than 9:1, more preferably from 5:1 to 1:1, more preferably from 4:1 to 2:1.

2. The composition according to any preceding claims, further comprising one or more co-proteins, wherein the co-proteins have at least 80%, preferably at least 85%, preferably at least 90%, preferably at least 95%, preferably at least 98% or even 100% amino acid identity as calculated over the entire length of the sequence aligned against the entire length of at least one reference sequence selected from the group consisting of: *Engystomops pustulosus* Ep-Rsn1 (SEQ ID NO: 5), *Engystomops pustulosus* Ep-Rsn3 (SEQ ID NO: 6), *Engystomops pustulosus* Ep-Rsn4 (SEQ ID NO: 7), *Engystomops pustulosus* Ep-Rsn5 (SEQ ID NO: 8), and *Engystomops pustulosus* Ep-Rsn6 (SEQ ID NO: 9); and mixtures thereof, preferably *Engystomops pustulosus* Ep-Rsn3 (SEQ ID NO: 6) and *Engystomops pustulosus* Ep-Rsn5 (SEQ ID NO: 8).

3. The composition according to any preceding claims, further comprising one or more carbohydrates selected from the group comprising O-glycan, N-glycan, and mixtures thereof.

4. The composition according to any preceding claims, wherein the surface active proteins are present in an amount

of from 0.0001 wt% to 5 wt%, preferably from 0.01 wt% to 1 wt%, by weight of the cleaning composition, based on active protein.

**5.** The composition according to any preceding claims, wherein the surfactant system is present in an amount of from 1 wt% to 60 wt%, preferably from 5 wt% to 50 wt%, by weight of the cleaning composition.

**6.** The composition according to any preceding claims, wherein the co-surfactants are selected from the group consisting of amphoteric surfactant, zwitterionic surfactant, and mixtures thereof, preferably the amphoteric surfactant is amine oxide surfactant and the zwitterionic surfactant is betaine surfactant.

**7.** The composition according to any preceding claims, further comprising a chelant, preferably selected from the group comprising carboxylate chelants, amino carboxylate chelants, amino phosphonate chelants, and mixtures thereof, preferably selected from the group of MGDA (methylglycine-N,N-diacetic acid), GLDA (glutamic-N,N-diacetic acid), and mixtures thereof.

**8.** The composition according to any preceding claims, further comprising one or more enzymes selected from the group consisting of amylases, lipases, proteases, cellulases, lipoxygenases, diol synthases, and mixtures thereof.

**9.** The composition according to any preceding claims, wherein the anionic surfactants are selected from the group consisting of: alkyl sulfates, alkyl alkoxy sulfates, alkyl benzene sulfonates, paraffin sulfonates, and mixtures thereof, preferably a mixture of alkyl sulfates and alkyl ethoxy sulfates.

**10.** The composition according to any preceding claims, wherein the anionic surfactants are a mixture of alkyl sulfates and alkyl alkoxy sulfates, wherein the co-surfactants are alkyl dimethyl amine oxides, and wherein the weight ratio of the anionic surfactants to the co-surfactants is from 4:1 to 2:1.

**11.** The composition according to any preceding claims, wherein the cleaning composition is a liquid manual dishwashing cleaning composition.

**12.** A method comprising contacting a surface preferably dishware with a cleaning composition according to any preceding claims, wherein the composition modifies the hydrophobicity of the surface as a result of the contacting step.

**13.** A method of promoting suds longevity or grease emulsification in a washing process for washing soiled articles, preferably dishware, comprising the steps of:

a) delivering the cleaning composition according to claims 1 to 11 to a volume of water to form a wash liquor; and
b) immersing the soiled articles into the wash liquor.

**14.** The method according to claims 13, wherein the surface active proteins are present at a concentration from 0.005 ppm to 60 ppm, preferably from 0.02 ppm to 12 ppm, based on active protein, in an aqueous wash liquor during the washing process.

**15.** Use of surface active proteins to provide improved suds longevity, improved grease emulsification or both in an aqueous wash liquor during a washing process, wherein the surface active proteins are selected from the group consisting of ranaspumins, latherins, and mixtures thereof, preferably the ranaspumins have at least 40%, preferably at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of at least one reference sequence selected from the group consisting of: *Engystomops pustulosus* Ep-Rsn2 (SEQ ID NO: 1), *Leptodactylus vastus* Lv-Rsn1 (SEQ ID NO: 2), *Leptodactylus fuscus* Lf-Rsn1 (SEQ ID NO: 3), and *Bufo gargarizans* Bg-Rsn (SEQ ID NO: 4), and the latherins have at least 40%, preferably at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of at least one reference sequence to *Equus caballus* latherin (SEQ ID NO: 10), preferably the surface active proteins are ranaspumins.

Europäisches Patentamt
European Patent Office
Office européen des brevets

# EUROPEAN SEARCH REPORT

Application Number
EP 18 17 8753

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | WO 2015/183320 A1 (HALLIBURTON ENERGY SERVICES INC [US]) 3 December 2015 (2015-12-03)<br>* paragraphs [0025], [0028] *<br>----- | 1,4,5,9, 11<br>2,3,6-8, 10 | INV. C11D3/38 C07K14/46 C07K14/47 |
| A | US 2009/110756 A1 (MCCRAY JR PAUL B [US] ET AL) 30 April 2009 (2009-04-30)<br>* paragraphs [0008], [0009], [0133], [0141] *<br>----- | 1-15 | |
| A | CHRISTOPHER HILL ET AL: "Foams: From nature to industry", ADVANCES IN COLLOID AND INTERFACE SCIENCE, vol. 247, 12 May 2017 (2017-05-12), pages 496-513, XP055467076, NL ISSN: 0001-8686, DOI: 10.1016/j.cis.2017.05.013<br>* abstract *<br>* page 505, right-hand column, last paragraph - left-hand column, paragraph 1 *<br>----- | 13-15 | TECHNICAL FIELDS SEARCHED (IPC)<br>C11D<br>C07K |
| A | SCHOR MARIEKE ET AL: "The Diverse Structures and Functions of Surfactant Proteins", TRENDS IN BIOCHEMICAL SCIENCES, ELSEVIER, AMSTERDAM, NL, vol. 41, no. 7, 27 May 2016 (2016-05-27), pages 610-620, XP029624822, ISSN: 0968-0004, DOI: 10.1016/J.TIBS.2016.04.009<br>* page 616, paragraph 2 - page 618, paragraph 2 *<br>----- | 13-15 | |

1

The present search report has been drawn up for all claims

| Place of search | Date of completion of the search | Examiner |
|---|---|---|
| The Hague | 22 October 2018 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 17 8753

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2018

| Patent document cited in search report | Publication date | Patent family member(s) | Publication date |
|---|---|---|---|
| WO 2015183320 A1 | 03-12-2015 | US 2017037301 A1 | 09-02-2017 |
| | | WO 2015183320 A1 | 03-12-2015 |
| US 2009110756 A1 | 30-04-2009 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 201219844 A
- WO 201219849 A
- WO 201219848 A
- US 3915903 A
- WO 2007135645 A


### Non-patent literature cited in the description


- **COOPER, A. et al.** *Colloids Surf., A: Physiochemical and Engineering Aspects,* 2017
- **SCHOR, M. et al.** *Trends Biochem. Sci.,* 2016, vol. 41 (7), 610-620
- **FLEMING, R. I. et al.** *Proc. R. Soc.,* 2009, vol. B 276 (1663), 1787-1795
- **BEELEY, J. G. et al.** *Biochem. J.,* 1986, vol. 235 (3), 645-650
- **PETERSEN TN. ; BRUNAK S. ; HEIJNE G. ; NIELSEN H.** *Nature Methods,* 2011, vol. 8, 785-786
- **NEEDLEMAN ; WUNSCH.** *J. Mol. Biol.,* 1970, vol. 48, 443-453
- **HENIKOFF S. ; HENIKOFF J.G.** *P.N.A.S. USA,* 1992, vol. 89, 10915-10919
- **ROBERT LAUGHLIN.** The Aqueous Phase Behaviour of Surfactants. Academic Press, 1994, 538-542